# EUROPEAN PATENT SPECIFICATION

(11) **EP 4 178 510 B1**
(45) Date of publication and mention of the grant of the patent: **28.08.2024**
(21) Application number: 21749246.1
(22) Date of filing: 09.07.2021
(51) Int. Cl.: A61F 13/00, A61B 5/103

(54) **PRESSURE SENSOR STRIP**
DRUCKSENSORLEISTE
BANDE DE CAPTEURS DE PRESSION

(30) Priority: 09.07.2020 GB 202010588
(43) Date of publication of application: 17.05.2023
(73) Proprietor: VeinSense Ltd, Wallcrouch Wadhurst East Sussex TN5 7JR (GB)
(72) Inventor: HARPIN, Arnold Peter Roscoe, Wallcrouch, Wadhurst East Sussex TN5 7JR (GB)
(74) Representative: Potter Clarkson
(86) International application number: PCT/GB2021/051762
(87) International publication number: WO 2022/008927

(56) References cited:
- WO-A1-2006/030405
- US-B1- 10 034 622

## Description

### Background of the invention

Leg ulcers are a life-limiting medical condition that responds poorly to current treatments. This causes long-term distress and possible immobility to the patent, and the long-term nature of the treatment and limited effectiveness makes it an expensive condition to treat.

The key to effective treatment is to bandage the leg in such a way that there is a pressure gradient up the leg, starting with a higher pressure (typically 5.2 kPa) at the ankle, reducing to 2.6 kPa at the top of the calf, just below the knee.

Conventional bandaging techniques attempt to achieve such a profile by wrapping a bandage at approximately uniform tension up the leg. The tension may be gauged by noting the amount of stretch in circles imprinted on the bandage. Theoretically, for a uniform tension, the pressure exerted by a bandage on a cylindrical structure (such as a leg) is inversely proportional to the radius of curvature (based on Laplace's Law). This, and the 'experience' of the applier of the bandage, is used to produce what is hoped is the correct profile. However typically the pressures exerted in this way are not as expected (often higher) and worse, the profile is not uniform. This could make the condition worse as a high spot in the pressure up the leg would tend to inhibit the desired flow of blood and other fluids up the leg, possibly even causing more serious conditions. In addition, the high pressure and non-uniformity of the pressure gradient causes unnecessary patient discomfort which results in a low uptake; patients tend to remove the bandage on comfort grounds or even re-fit themselves for comfort, thus completely invalidating the treatment.

Ideally a pressure sensor can be deployed, which is fitted between the leg and bandage to measure the pressures at points up the leg (typically about 5 or 6). However, it is important that the pressure sensor itself does not cause an erroneous pressure reading by perturbing the pressure where it sits. Typically, inserting an object between the leg and bandage causes a local (possibly dangerous, and definitely a contributor to inaccuracy) elevation in pressure. Attempts to mitigate these difficulties have included making small thin sensors, but these tend to be expensive, relying on such technologies as fibre gratings and complex interrogation schemes. An added difficulty is that the pressures are quite low, and the sensor needs to be immune to shear and twisting effects.

WO2017/174984 discloses a sensor comprising first and second layers, separated by an elastic spacer. The separation of the first and second layers is determined by capacitive or optical means. This gives an indication of the pressure applied to the top and bottom layers. The sensor is essentially rectangular in cross section.

WO 2006/030405 A1 discloses a transducer apparatus having a transducer which lies between a bandage and a patient's leg, for example. An active part has a sensor element comprising an array of sub-elements of trapezoidal shape in cross-section. The sensor element is a dielectric between electrodes on substrates. The assembly is enveloped by encapsulant polymer films.

### Summary of the invention

The present invention is based on the realisation that the pressure sensors of the type described in the prior art exerts a significant local pressure. That can be rationalised, in retrospect (as explained below), and can be mitigated to a surprising extent by a sensor strip according to the invention: this sensor strip comprises an elongate member that is segmented; the segments being defined by longitudinal and transverse grooves, such that the adjacent segments are hinged together; the longitudinal grooves define a central column of segments and outer columns of segments; the segments in the outermost columns are tapered outwardly; and a pressure sensitive sensor is mounted in each of a plurality of the segments in the central column.

A sensor of the invention can be used by introducing it under a bandage and recording the pressure at each of the pressure-sensitive sensors.

### Description of the figures

An example of the present invention will now be described in detail, with reference to the accompanying figures, in which:
Figure 1(a) shows a simplified plan view of an example of an elongate segmented sensor strip in accordance with the present invention;
Figure 1(b) shows a cross sectional view along the line A-A in Figure 1(a);
Figure 1(c) shows cross sectional view along the line B-B in Figure 1(a) to illustrate the hinging and separation of the segments of the sensor strip in use;
Figure 2 is a schematic illustration of the elongate sensor strip partially wrapped around the circumference of a section of a leg;
Figures 3 illustrates the geometry of a segmented sensor strip in accordance with the present invention;
Figure 4 is for comparative purposes and illustrates alternative sensor geometry; and,
Figures 5(a) - 5(d) show plan and cross-sectional views of an example of a sensor strip in accordance with the present invention.

### Description of the invention

In preferred embodiments of the present invention, we measure the displacement of a flexible elongate sensor strip using optical proximity sensors mounted to the strip. Capacitive sensors may also be used. Such a sensor strip is shaped in such a way that it does not significantly alter the pressure exerted by a bandage, in that the sensor strip conforms to the curvature of the leg where it is fitted. In addition, the sensor strip is tapered to avoid any pressure enhancement at the edge of the sensor strip.

In a preferred embodiment, the sensor strip is from 300 to 450 mm long (typically 380 mm long) and 10-15 mm wide. By virtue of the tapering structure and the segmentation to follow the radius of curvature, the sensor strip can be of 2-4 mm thick, which allows cost-effective sensing techniques (the sensor strip will have a short service life, typically days per patient).

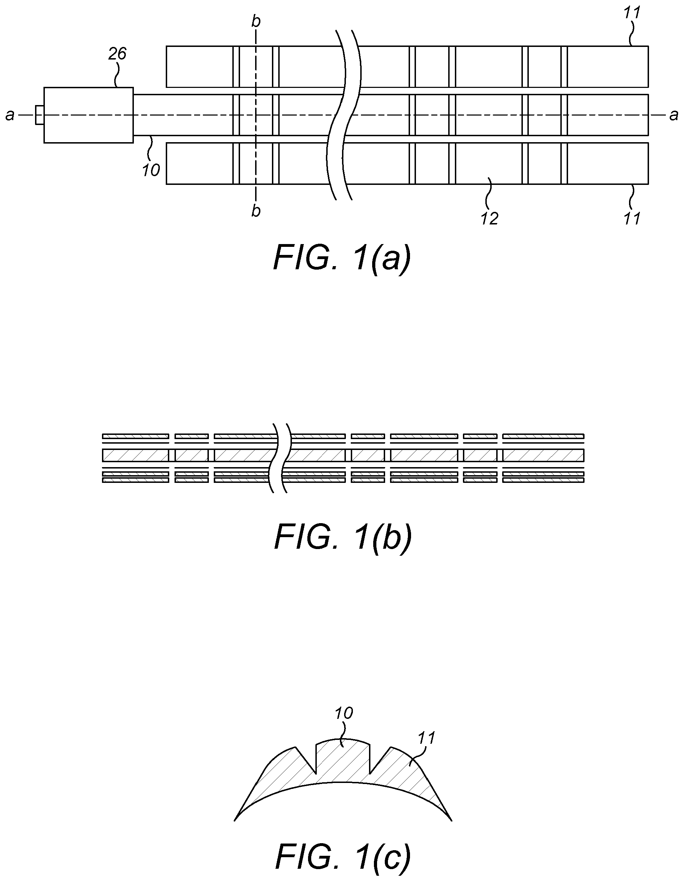

Referring now to the Figures, a sensor strip of the type shown in Fig.1 can be used by mounting it along a calf with an electrical connector 26 placed just below the knee in typical operation.

The segmentation of the sensor strip allows it to follow the curvature of the leg at any point. The sensor strip comprises a central column 10 and outer columns 11 and each of the segments 12 may have a rigid top and bottom surface.

In preferred embodiments, the widths of the segments 12 are typically 10 mm, e.g., not more than 20 mm, and as low as 5 mm. The thickness of the sensor strip should be as low as practically possible, but 1-5 mm is a workable range. This helps to isolate the sensor cell from shearing forces that would also tend to stretch the upper surface of the sensor strip relative to the lower surface, and such a bending effect would register as a false pressure. This geometry ensures the sensor strip only responds to the pressure exerted by the bandage, and that this pressure is communicated directly to the leg.

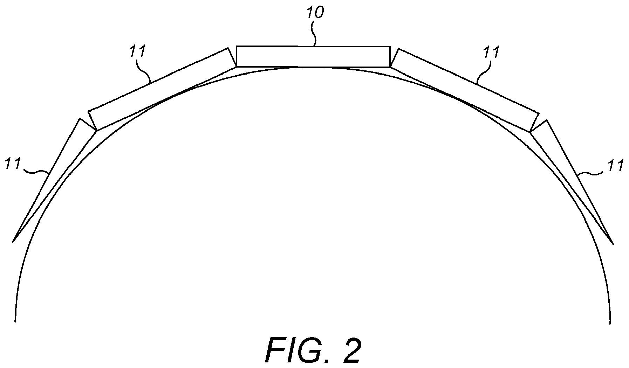

In some embodiments, there is one outer column 11 either side of the central column 10 as shown in Fig. 1, but it is possible and may enhance performance if there are two outer columns 11 on each side of central column 10 - i.e., a total of 5 segments as shown in Fig.2. Typically, the length of each segment is around 50 mm. This may be dictated by the compliance of the sensor strip and leg geometry and might be as low as 10 mm. 50-60 mm is often the largest practical size.

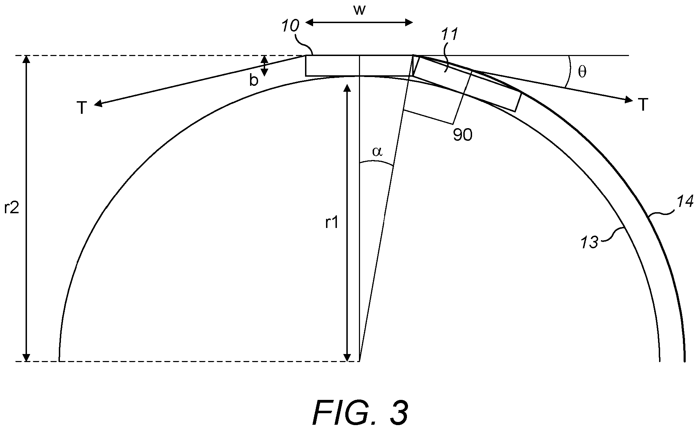

Figure 3 shows how the sensor strip geometry works. For clarity, only one outer column 11 is shown to the right of the central column 10, as opposed to a sensor strip construction in accordance with the present invention where there is an outer column either side. The drawing shows in addition a mandrel 13 and a bandage 14.

It can be shown that the pressure exerted on the flat upper surface of the sensor's central column 10 will be the same as if the sensor strip was not there (other than a small change of a few percent due to the fact that the sensor thickness of typically 3 mm adds to the effective radius of the leg which might be 35 mm or more), if the bandage leaves the edge of the central column 10 at the correct angle θ, where this angle is the angle of the tangent to the curvature of the bandage shown by the bold curved line. The force exerted on the sensor strip and hence the leg is given by the equation *F* = *T*sin*θ*. Where F is the downward force and T is the tension in the bandage.

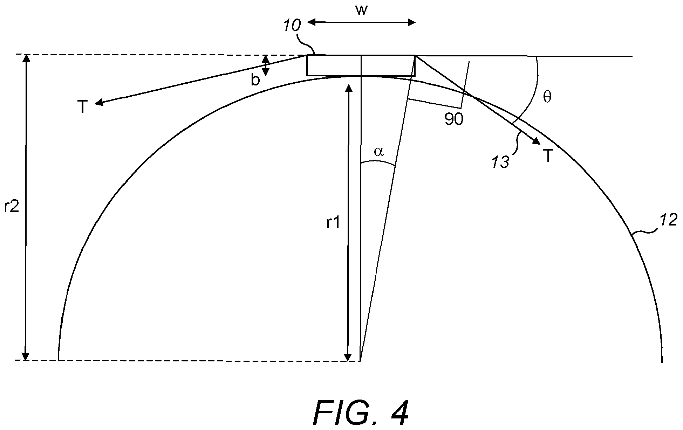

It will be appreciated that if the outer column 11 were not there the sensor strip would read a greatly exaggerated pressure, and this pressure would be exerted locally on the leg, as can be seen from the comparative example in Fig. 4. The pressure could be increased by as much as a factor of 5.

The pivot and segmentation need to ensure the bandage follows to within a certain accuracy the same path as it would if the sensor strip was a continuous (unsegmented) flexible layer, as opposed to rigid segments joined together. It can be shown geometrically and theoretically that as long as the gap between segments is small (< 1 mm) and the column width is <15mm that the error in pressure is <10%. Similar considerations apply to the design of a one-piece segmented structure.

The segmentation allows the use of a relatively rigid material to form the sensor strip of a only a few mm in thickness, which enables cost-effective mass manufacturable sensor strips.

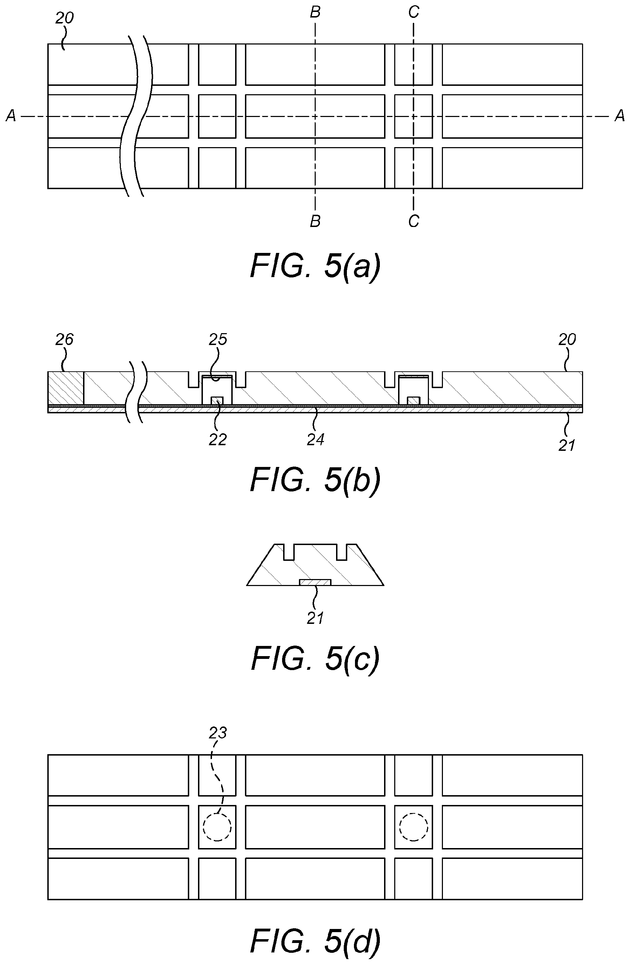

Fig. 5(a) shows a sensor strip of the present invention formed by mounting a rubber strip 20 on a flexible PCB substrate 21. Fig. 5(b) is a section A-A along Fig. 5(a), which shows in more detail a connector 26, optical proximity sensors 22, each formed in cavity 23, with an (optional) stiffener 24 and reflectors 25. Fig. 5(c) is a section along B-B in Fig. 5(a). The position of each cavity 23 is illustrated in Fig. 5(d).

Preferably, the upper surface of the rubber strip 20 is textured, or a layer of a suitable material is laid on top, in order to enhance contact reproducibility.

In some embodiments, a sensor strip of the present invention comprises a one-piece rubber strip or similar structure with a grooved surface to provide the segmentation, below which is attached a flexible PCB to route the signals to the electrical connector and to mount the optical proximity detectors.

Fig. 5(b) shows the flexible PCB 21 running the length of the sensor strip. The sensor cavity 23 is a recess, preferably a circular recess, formed in the rubber strip 20, the internal top surface of which may be coated with suitable reflective material 25, preferably a diffuse Lambertian reflector such as a suitable white paint printed onto the rubber.

In Fig. 5, the top surface of each sensor cavity is shown as square, but circular is an option. A square may be used as it presents a uniform edge for the bandage to land on, given the curved geometry the sensor strip is designed to be used on e.g., a human leg.

The contact area with the bandage should be constant and preferably as close to 100% of the apparent contact area as possible. Black, Closed-cell, Firm Grade Neoprene Foam is a suitable material. The spring constant of the sensor strip and therefore the full-scale excursion of the sensor strip is determined largely by the Youngs Modulus of the rubber foam (HT800), but depends on the area of the recess 23. Optionally, voids could be moulded into the rubber around the sensor cavity to further reduce the area of rubber between the top and bottom of the sensor strip and hence the effective Youngs Modulus of the rubber. There may be an additional rubber layer under the flexible PCB and the stiffeners for encapsulation and to present a uniform surface to the leg.

Another feature is that low-cost phototransistors may be used in the design. However, they have a significant temperature drift which adversely affects the accuracy of the sensors. This can be mitigated by providing an extra proximity detector either next to each sensor or in the centre of the strip, which is set in a cavity that does not respond to pressure. Thus, it will only respond to temperature and this reading can be used to null out any temperature drift. It might be thought that an extra proximity sensor needs to be provided for each sensor but as the sensor will be used under a bandage it is likely it will be at a uniform temperature and so only one compensation device needs to be provided.

The flexible PCB substrate 21 contains the proximity sensors 22 which preferably comprise an LED mounted next to a phototransistor, as is well known to produce a proximity detector. Over a distance of about 1 mm the divergence of the light from the LED ensures that the amount reflected back to the phototransistor varies with distance. The LED and phototransistor are both mounted on the flexible PCB substrate 21 so that the optical axes are parallel and about 1 mm apart, with a barrier to prevent cross talk.

It will be appreciated that other optical configurations are possible, including lenses on the LED or phototransistor to modify the light received vs. distance function. At present, to eliminate errors due to skew or the reflector not being quite parallel to the base of the sensor, we use a Lambertian reflector which has a low angular dependence compared to a specular reflector. A corner cube type reflector could be used instead to avoid skew effects completely. It will also be appreciated that the LED/phototransistor combination could operate at a non-visible wavelength such as infra-red as this allows the phototransistor to incorporate a filter to reduce ambient light sensitivity, which would otherwise affect the readings. In practice, the sensor is enclosed but the low signal levels mean that even low levels of ambient light can perturb the readings. A standard technique to eliminate this effect further is used in that the LED is pulsed on and off (at about 1 KHz). The readings from the phototransistor are taken at LED OFF and LED ON states and this allows subtraction of any offset due to ambient light. The preferred pulsing at 1 KHz also avoids effects due to noise pickup which tends to be at mains frequency of 50 Hz.

The electrical connector of the sensor strip can be coupled to a separate interrogator unit (not shown), typically a small electronics box which could be belt worn by a patient. The interrogator unit houses electronics that are configured to pulse the LEDs and receive the signals from the phototransistors and amplify them using transimpedance amplifiers. The amplifiers are preferably rolled off at about 50 KHz as this allows enough bandwidth whilst keeping noise relatively low. The signal for LED ON and LED OFF is sampled by a microcontroller, but a delay is introduced so the signal can settle before it is sampled. This avoids any effects due to bandwidth limitations affecting the signal. Typically, 100-1000 samples are taken and then averaged to derive a reading.

Other modulation schemes such as a sinusoidal modulation could be used.

The individual sensors are generally calibrated hydrostatically, as this avoids contact area issues and is considered a gold standard for pressure calibration. A sensor typically will have a transfer function of output voltage vs pressure and this function is linear with both an offset due to manufacturing tolerances and a quadratic term due to the non-linearity of the proximity detector transfer function. The calibration data in the form of the terms *a,b,c* in the polynomial *ax*² + *bx* + *c* are stored in a non-volatile memory chip (not shown) integrated on the flexible PCB strip. These data are then read by the microcontroller in the interrogation unit and used to calculate an actual pressure reading from the signals from the individual sensors. A temperature correction can also be applied. It should be noted that the individual sensors are not expected to drift excessively during storage and therefore if the voltage at zero pressure is different from the value at calibration (which can also be stored on the memory chip) by too great a margin the sensor will be flagged as faulty. Similarly, in a preferred embodiment the interrogator unit is configured to measure the current drawn by the LEDs and if this is different will be flagged as an error. It would be possible to pinpoint the current change associated with one sensor on the sensor strip failing and to continue using the others (typically 5) on the sensor strip.

Other data are stored on the memory chip for each sensor such as batch number, date of manufacture, date of calibration, number of uses or whether first time use or not (for single use sensors).

In a preferred embodiment, the interrogator unit provided is able to communicate recorded measurements via a USB interface to PC, tablet etc, or to a Bluetooth-enabled device for display on a graphical user interface (GUI). A typical user interface is a histogram type display showing the pressure graded up the leg.

## Claims

1. A sensor strip comprising an elongate member that is segmented, the segments (12) being defined by continuous longitudinal and transverse grooves, such that adjacent segments (12) are hinged together, wherein the longitudinal grooves define a central column (20) of segments (12) and outer columns (11) of segments, wherein the segments (12) in the outermost columns (11) are tapered outwardly, and **characterised in that** a pressure-sensitive sensor is mounted in a plurality of the segments (12) in the central column (11).

2. A sensor strip according to claim 1, which comprises at least 3 pressure-sensitive sensors.

3. A sensor strip according to claim 1 or claim 2, which comprises up to 6 pressure-sensitive sensors.

4. A sensor strip according to any preceding claim, wherein the pressure-sensitive sensors are mounted in non-adjacent segments (12).

5. A sensor strip according to any preceding claim, which comprises at least 2 outer columns (11) to both sides of the central column (20).

6. A sensor strip according to any preceding claim, which additionally comprises a reference, non-pressure-sensitive sensor.

7. A sensor strip according to any preceding claim, wherein the base of each segment (12) in which a pressure-sensitive sensor is mounted, is rigid.

8. A sensor strip according to any preceding claim, wherein each of the segments (12) is formed from a rigid material.

9. A sensor strip according to any preceding claim, in which the segments (12) are formed from a rubber material, preferably a NEPORENE foam, and more preferably a contiguous sheet of segmented material.

10. A sensor strip according to any preceding claim, further comprising an electrical connector (26) to couple data signals from the pressure-sensitive sensors to a remotely coupled interrogator unit for data processing.

11. A method of testing the pressure under a bandage (14), which comprises introducing a sensor strip according to any preceding claim under the bandage (14), and recording the pressure at each of the pressure-sensitive sensors.

## Patentansprüche

1. Sensorstreifen, umfassend ein längliches Element, welches segmentiert ist, wobei die Segmente (12) durch fortlaufende Längs- und Querrillen definiert sind, sodass benachbarte Segmente (12) aneinander angelenkt sind, wobei die Längsrillen eine zentrale Säule (20) von Segmenten (12) und äußere Säulen (11) von Segmenten definieren, wobei die Segmente (12) in den äußersten Säulen (11) nach außen abgeschrägt sind, und **dadurch gekennzeichnet, dass** ein druckempfindlicher Sensor in einer Vielzahl von Segmenten (12) in der zentralen Säule (11) montiert ist.

2. Sensorstreifen nach Anspruch 1, welcher zumindest 3 druckempfindliche Sensoren umfasst.

3. Sensorstreifen nach Anspruch 1 oder Anspruch 2, welcher bis zu 6 druckempfindliche Sensoren umfasst.

4. Sensorstreifen nach einem vorstehenden Anspruch, wobei die druckempfindlichen Sensoren in nicht benachbarten Segmenten (12) montiert sind.

5. Sensorstreifen nach einem vorstehenden Anspruch, welcher zumindest 2 äußere Säulen (11) zu beiden Seiten der zentralen Säule (20) umfasst.

6. Sensorstreifen nach einem vorstehenden Anspruch, welcher zusätzlich einen nicht druckempfindlichen Referenzsensor umfasst.

7. Sensorstreifen nach einem vorstehenden Anspruch, wobei die Basis jedes Segments (12), in welchem ein druckempfindlicher Sensor montiert ist, starr ist.

8. Sensorstreifen nach einem vorstehenden Anspruch, wobei jedes der Segmente (12) aus einem starren Material gebildet ist.

9. Sensorstreifen nach einem vorstehenden Anspruch, in welchem die Segmente (12) aus einem Gummimaterial, bevorzugt einem NEOPRENE-Schaumstoff, und bevorzugter einem anliegenden Blatt von segmentiertem Material gebildet sind.

10. Sensorstreifen nach einem vorstehenden Anspruch, weiter einen elektrischen Steckverbinder (26) umfassend, um Datensignale aus den druckempfindlichen Sensoren mit einer entfernt gekoppelten Abfrageeinheit zur Datenverarbeitung zu koppeln.

11. Verfahren zum Testen des Drucks unter einem Verband (14), welches Einführen eines Sensorstreifen nach einem vorstehenden Anspruch unter den Verband (14) und Aufzeichnen des Drucks an jedem der druckempfindlichen Sensoren umfasst.

## Revendications

1. Bande de détection comprenant un élément allongé qui est segmenté, les segments (12) étant définis par des rainures continues longitudinales et transversales, de sorte que des segments adjacents (12) soient articulés ensemble, dans laquelle les rainures longitudinales définissent une colonne centrale (20) de segments (12) et des colonnes extérieures (11) de segments, dans laquelle les segments (12) dans les colonnes (11) les plus à l'extérieur sont effilés vers l'extérieur, et **caractérisée en ce que**
un capteur sensible à la pression est monté dans une pluralité des segments (12) dans la colonne centrale (11).

2. Bande de détection selon la revendication 1, qui comprend au moins 3 capteurs sensibles à la pression.

3. Bande de détection selon la revendication 1 ou la revendication 2, qui comprend jusqu'à 6 capteurs sensibles à la pression.

4. Bande de détection selon une quelconque revendication précédente, dans laquelle les capteurs sensibles à la pression sont montés dans des segments (12) non adjacents.

5. Bande de détection selon une quelconque revendication précédente, qui comprend au moins 2 colonnes extérieures (11) sur les deux côtés de la colonne centrale (20).

6. Bande de détection selon une quelconque revendication précédente, qui comprend de plus un capteur de référence non sensible à la pression.

7. Bande de détection selon une quelconque revendication précédente, dans laquelle la base de chaque segment (12) dans lequel est monté un capteur sensible à la pression est rigide.

8. Bande de détection selon une quelconque revendication précédente, dans laquelle chacun des segments (12) est formé à partir d'un matériau rigide.

9. Bande de détection selon une quelconque revendication précédente, dans laquelle les segments (12) sont formés à partir d'un matériau de caoutchouc, de préférence d'une mousse NÉOPRÈNE et, de manière davantage préférée, d'une feuille contiguë de matériau segmenté.

10. Bande de détection selon une quelconque revendication précédente, comprenant en outre un connecteur électrique (26) pour coupler des signaux de données provenant des capteurs sensibles à la pression à une unité d'interrogation couplée à distance pour un traitement de données.

11. Procédé de test de la pression sous un pansement (14), qui comprend l'introduction d'une bande de détection selon une quelconque revendication précédente sous le pansement (14) et l'enregistrement de la pression au niveau de chacun des capteurs sensibles à la pression.
